# EUROPEAN PATENT APPLICATION

(11) **EP 4 368 201 A1**
(43) Date of publication of application: **15.05.2024**
(21) Application number: 22837768.5
(22) Date of filing: 08.07.2022
(51) Int. Cl.: A61K 39/106, A61P 31/04, A23K 10/16, A23K 50/80, A01K 61/59, C12N 1/20

(54) **COMPOSITION FOR REARING ORGANISM BELONGING TO ORDER DECAPODA AND COMPOSITION FOR PREVENTING OR TREATING INFECTION IN DECAPODS**

(30) Priority: 09.07.2021 JP 2021114165
(71) Applicant: Sumitomo Chemical Company, Limited, Tokyo 103-6020 (JP)
(72) Inventor: AOKI, Mikio, Osaka-shi, Osaka 554-8558 (JP); MIKATA, Kazuki, Osaka-shi, Osaka 554-8558 (JP); KAI, Toshihiro, Osaka-shi, Osaka 554-8558 (JP); HIRONO, Ikuo, Tokyo 108-8477 (JP); KONDO, Hidehiro, Tokyo 108-8477 (JP); MATSUMOTO, Sana, Tokyo 108-8477 (JP)
(74) Representative: Vossius & Partner Patentanwälte Rechtsanwälte mbB
(86) International application number: PCT/JP2022/027145
(87) International publication number: WO 2023/282354

(57) **Abstract**

Provided are: a composition for rearing an organism belonging to the order Decapoda, comprising bacterial cells or a cell culture product of at least one bacterium selected from NITE-BP-03199, NITE-BP-03200, and NITE-BP-03201, or an extract thereof; and a method for rearing an organism belonging to the order Decapoda using the same. Provided are: a composition for preventing or treating an infection caused by a *Vibrio* bacterium in a decapod, comprising bacterial cells or a cell culture product of at least one bacterium selected from NITE-BP-03199, NITE-BP-03200, and NITE-BP-03201, or an extract thereof; and a method for preventing or treating an infection in a decapod using the same.

## Description

### TECHNICAL FILED

The present invention relates to a composition for rearing an organism belonging to the order Decapoda, and a composition for preventing or treating an infection in a decapod. The present invention also relates to a method for rearing an organism belonging to the order Decapoda with any of the compositions.

### BACKGROUND ART

In recent years, early mortality syndrome/acute hepatopancreatic necrosis disease (hereinafter, also referred to as "EMS/AHPND") is causing more and more damage in shrimp aquaculture farms. Outbreaks of EMS/AHPND have been reported in China, Thailand, Vietnam, Malaysia, Mexico, and so on, and in 2020 such an outbreak was also reported in Japan. An outbreak of EMS/AHPND in an aquaculture pond causes massive, rapid death of shrimp in 20 to 30 days, and the mortality rate is close to 100%. Thus, shrimp aquaculture industries in some countries are facing dangerous situations. It has been revealed that *Vibrio parahaemolyticus* of special type is a primary causative bacterium for EMS/AHPND. Infection test by a PCR method has been carried out for juvenile shrimp to control EMS/AHPND. However, some juvenile shrimp determined to be negative in the test may undergo the onset of EMS/AHPND, and it is difficult to prevent EMS/AHPND by infection test alone. Most developed countries prohibit importing shrimp farmed with an antibiotic, and preventing or treating EMS/AHPND by administering an antibiotic is also restricted.

Methods for reducing damage to be caused by bacterial infection in aquaculture of shrimp have been previously developed. For example, Japanese Patent Laying-Open No. 2015-137254 (PTL 1) discloses a vaccine composition that induces immunity against *Vibrio* bacteria in shrimp. However, a vaccine containing *Vibrio parahaemolyticus,* which is a causative bacterium for EMS/AHPND, has not been developed yet, and the preventive and therapeutic effects of the vaccine composition for EMS/AHPND are unknown. WO 2019/059027 (PTL 2) discloses preventing/treating EMS/AHPND by allowing an organism belonging to the order Decapoda to ingest a composition containing 5-aminolevulinic acid for oral administration to promote the growth. Sudarat Chomwong et al. Developmental and Comparative Immunology, Vol. 89 Page. 54-65 (2018) (NPL 1) has reported a method of activating the defense system to increase the resistance to EMS/AHPND with the *Lactobacillus plantarum* SGLAB01 strain and *Lactococcus lactis* SGLAB02 strain derived from the shrimp gut, or a feed supplemented with probiotics (living bacteria) of the combination. The growth promoter and probiotics indirectly increase the resistance to EMS/AHPND by acting on the defense system of shrimp, without exhibiting direct antibacterial activity to causative bacteria for EMS/AHPND.

### CITATION LIST

### PATENT LITERATURE

PTL 1: Japanese Patent Laying-Open No. 2015-137254
PTL 2: WO 2019/059027

### NON PATENT LITERATURE

NPL 1: Sudarat Chomwong et al. Development and Comparative Immunology, vol. 89, Page. 54-65 (2018)

### SUMMARY OF INVENTION

### TECHNICAL PROBLEM

An object of the present invention is to provide a method for inhibiting infections in decapods with less impact on the environment and less toxicity to humans.

### SOLUTION TO PROBLEM

The present invention relates to items exemplified in the following.
[1] A composition for rearing an organism belonging to the order Decapoda, containing bacterial cells or a cell culture product of at least one bacterium selected from NITE-BP-03199, NITE-BP-03200, and NITE-BP-03201, or an extract thereof.
[2] A composition for preventing or treating an infection caused by a *Vibrio* bacterium in a decapod, containing bacterial cells or a cell culture product of at least one bacterium selected from NITE-BP-03199, NITE-BP-03200, and NITE-BP-03201, or an extract thereof.
[3] The composition according to [2], wherein the infection in a decapod includes early mortality syndrome/acute hepatopancreatic necrosis disease.
[4] The composition according to any of [1] to [3], wherein the composition is a feed or feed additive.
[5] The composition according to any of [1] to [3], wherein the composition is rearing water or a rearing water additive.
[6] The composition according to any of [1] to [5], wherein the order Decapoda includes the family Penaeidae.
[7] A method for rearing an organism belonging to the order Decapoda, comprising allowing the organism belonging to the order Decapoda to ingest bacterial cells or a cell culture product of at least one bacterium selected from NITE-BP-03199, NITE-BP-03200, and NITE-BP-03201, or an extract thereof.
[8] A method for rearing an organism belonging to the order Decapoda, comprising soaking the organism belonging to the order Decapoda in rearing water containing bacterial cells or a cell culture product of at least one bacterium selected from NITE-BP-03199, NITE-BP-03200, and NITE-BP-03201, or an extract thereof.
[9] A method for preventing or treating an infection caused by a *Vibrio* bacterium in a decapod, comprising allowing an organism belonging to the order Decapoda to ingest bacterial cells or a cell culture product of at least one bacterium selected from NITE-BP-03199, NITE-BP-03200, and NITE-BP-03201, or an extract thereof.
[10] A method for preventing or treating an infection caused by a *Vibrio* bacterium in a decapod, comprising soaking an organism belonging to the order Decapoda in rearing water containing bacterial cells or a cell culture product of at least one bacterium selected from NITE-BP-03199, NITE-BP-03200, and NITE-BP-03201, or an extract thereof.
[11] The method according to [9] or [10], wherein the infection in a decapod includes early mortality syndrome/acute hepatopancreatic necrosis disease.
[12] The method according to any of [7] to [11], wherein the order Decapoda includes the family Penaeidae.
[13] A method for rearing an organism belonging to the order Decapoda, comprising providing the composition according to any of [1] to [6] by dropping the composition into water to rear the organism belonging to the order Decapoda.
[14] A growth-inhibiting or bactericidal method for a *Vibrio* bacterium, comprising bringing bacterial cells or a cell culture product of at least one bacterium selected from NITE-BP-03199, NITE-BP-03200, and NITE-BP-03201, or an extract thereof into contact with the *Vibrio* bacterium.

### ADVANTAGEOUS EFFECTS OF INVENTION

The present invention can inhibit infections in decapods with less impact on the environment and less toxicity to humans. In addition, the present invention can inhibit infections by exhibiting direct antibacterial activity to causative bacteria for infections in decapods.

### BRIEF DESCRIPTION OF DRAWINGS

Fig. 1 shows photographs showing (A) the colony shape of and (B) a result of Gram staining of NITE BP-03199 in Experiment 1.
Fig. 2 shows photographs showing (A) the colony shape of and (B) a result of Gram staining of NITE BP-03200 in Experiment 2.
Fig. 3 shows photographs showing (A) the colony shape of and (B) a result of Gram staining of NITE BP-03201 in Experiment 3.
Fig. 4 shows a diagram illustrating test for evaluating antibacterial activity to *Vibrio* bacteria (plate culture) in Experiment 4.
Fig. 5 shows a graph representing the inhibiting effects of NITE BP-03200 and NITE BP-03201 on an infection caused by a *Vibrio* bacterium in decapods in Experiment 7.
Fig. 6 shows a graph representing the inhibiting effect of NITE BP-03201 on an infection caused by a *Vibrio* bacterium in decapods in Experiment 8.

### DESCRIPTION OF EMBODIMENTS

Hereinafter, modes of implementation of the present invention will be described in detail. The present invention is not limited to the following embodiments. Herein, each expression in the format "A to B" indicates the upper limit and lower limit of a range (i.e., A or more and B or less), and if a unit is shown only for B but not for A, the units of A and B are the same.

### [Composition for rearing organism belonging to order Decapoda]

A composition for rearing an organism belonging to the order Decapoda according to an embodiment of the present invention contains bacterial cells or a cell culture product of at least one bacterium selected from NITE-BP-03199, NITE-BP-03200, and NITE-BP-03201, or an extract thereof. The composition for rearing an organism belonging to the order Decapoda according to the present invention can prevent or treat infections caused by *Vibrio* bacteria in organisms belonging to the order Decapoda.

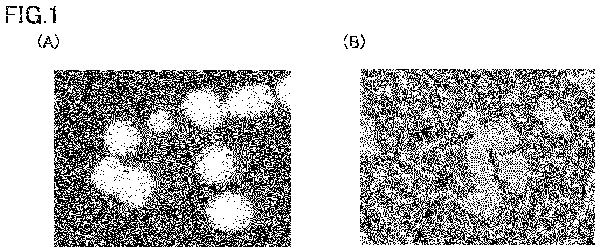

NITE-BP-03199, NITE-BP-03200, and NITE-BP-03201 are bacteria internationally deposited in the National Institute of Technology and Evaluation NITE Patent Microorganisms Depositary (NPMD, Address: Room 122, 2-5-8, Kazusakamatari, Kisarazu, Chiba, Japan 292-0818) in accordance with the Budapest Treaty under Accession Number: NITE BP-03199 (Original date of deposit: April 9, 2020), Accession Number: NITE BP-03200 (Original date of deposit: April 9, 2020), and Accession Number: NITE BP-03201 (Original date of deposit: April 9, 2020), respectively. NITE-BP-03199, NITE-BP-03200, and NITE-BP-03201 are each a bacterium belonging to *Lactobacillus plantarum,* which is one of lactic acid bacteria. The bacteriological characteristics of the bacterial strains are shown in Table 1 to Table 6 and Fig. 1 to Fig. 3 presented later.

NITE-BP-03199, NITE-BP-03200, and NITE-BP-03201 are present in the natural environment, and hence they are inferred to be highly safe in using for rearing of shrimp. Each of NITE-BP-03199, NITE-BP-03200, and NITE-BP-03201 may be an isolated bacterium.

The composition for rearing an organism belonging to the order Decapoda according to the present invention contains bacterial cells or a cell culture product of any of the lactic acid bacteria, or an extract thereof. The bacterial cells may be cells isolated from the environment, or cultured one. The bacterial cells may be dead bacterial cells or living bacterial cells. The bacterial cells may be cells in a culture solution, a buffer solution, or the like, or in a product obtained by concentrating the culture solution, buffer solution, or the like to remove the liquid or a freeze-dried product thereof, or in a frozen stock.

The cell culture product may contain secretions, metabolites, and others from the bacterium. In the cell culture product, peptides, proteins, saccharides, enzymes, and organic acids produced by the bacterium, and medium (liquid medium or solid medium) containing any of them are contained. The cell culture product may be the supernatant in which the bacterium was cultured. The culture supernatant can be obtained, for example, by removing the bacterium from the liquid medium in which the bacterium was cultured through centrifugation, filtration operations, or the like.

NITE-BP-03199, NITE-BP-03200, and NITE-BP-03201 can be cultured according to a common culture method for lactic acid bacteria. A representative culture method is, for example, a method of culturing with MRS (de Man, Rogosa and Sharpe) liquid medium or MRS agar medium at a temperature of 30°C.

The extract of the bacterial cells or the cell culture product is prepared in such a manner that the antibacterial activity of the bacterial cells or the cell culture product of the lactic acid bacterium to *Vibrio* bacteria is not lost. The extract can be obtained, for example, by treating the bacterial cells or the cell culture product of the bacterium through ultrasonic homogenization, bead grinding, freeze-thawing, or chemical dissolution. The extract may be obtained, for example, by performing salting-out, ultrafiltration, ion-exchange chromatography, or liquid-phase extraction with organic solvent on the bacterial cells or the cell culture product. These operations can be performed in combination, as appropriate. The extract can contain bacterial cell fragments, nucleic acids, peptides, proteins, saccharides, and enzymes from the bacterium. Herein, the bacterial cells or the cell culture product of the bacterium, or the extract thereof is also referred to as the "bacterial cell preparation".

Herein, the order Decapoda, also referred to as decapods, is one of taxa for crustaceans. The order Decapoda includes shrimp, crabs, and hermit crabs. The order Decapoda includes the suborder Dendrobranchiata and the suborder Pleocyemata. The suborder Dendrobranchiata includes the family Sergestoidea, the family Penaeidae, the family Sicyoniidae, the family Solenoceridae, the family Aristeidae, and the family Benthesicymidae. The family Penaeidae includes giant tiger prawn (common name: black tiger) *(Penaeus monodon),* whiteleg shrimp (common name: *vannamei* shrimp) *(Litopenaeus vannamei*), fleshy prawn *(Fenneropenaeus chinensis),* kuruma prawn *(Marsupenaeus japonicus),* western king prawn *(Melicertus latisulcatus),* offshore greasyback prawn *(Metapenaeus ensis),* shiba shrimp *(Metapenaeusjoyneri),* green tail prawn *(Metapenaeus moyebi),* red rice prawn *(Metapenaeopsis barbata),* broad velvet shrimp *(Metapenaeopsis lata),* green tiger prawn *(Penaeus semisulcatus),* and southern rough shrimp (*Trachysalambria curvirostris*), and the like. The family Penaeidae includes many important species for the shrimp aquaculture industry. The suborder Pleocyemata includes the infraorder Stenopodidea, the infraorder Caridea, the infraorder Polychelida, the infraorder Achelata, the infraorder Astacidea, the infraorder Glypheidea, the infraorder Axiidea, the infraorder Gebiidea, the infraorder Anomura, and the infraorder Brachyura.

Each of the compositions for rearing an organism belonging to the order Decapoda may be a composition for oral administration. The composition for oral administration may be any composition to be orally administered to an organism belonging to the order Decapoda, without limitation. The composition for rearing an organism belonging to the order Decapoda may be a feed or feed additive for organisms belonging to the order Decapoda. The composition for oral administration may be administered to an environment in which an organism belonging to the order Decapoda is reared (e.g., rearing water) and orally ingested by the organism belonging to the order Decapoda reared in the environment, into which the lactic acid bacterial cell preparation is released by dissolution. The composition containing the lactic acid bacterial cell preparation for oral administration to organisms belonging to the order Decapoda can prevent or treat infections caused by *Vibrio* bacteria in an organism belonging to the order Decapoda to which the composition has been administered.

The feed for decapods may contain any component that is typically used for rearing or aquaculture of organisms belonging to the order Decapoda, and may be produced by any production method. An appropriate composition can be select for the feed for decapods, depending on the type and growth stage of the decapod. The feed for decapods may contain a protein source such as squid meal, krill meal, fish meal, soybean meal, and corn gluten meal, and a binder such as gluten and starch. The feed for decapods may contain a known carrier or additive acceptable for feeds, and may contain, for example, a medicament for aquatic organisms such as an erythromycin preparation, an ampicillin preparation, a praziquantel preparation, a lysozyme chloride preparation, an oxytetracycline hydrochloride preparation, a spiramycin preparation, a sodium nifurstyrenate preparation, a lincomycin hydrochloride preparation, a flumequine preparation, and a glutathione preparation, a nutritional supplement substance such as a vitamin such as vitamin C, vitamin B 1, vitamin A, vitamin D, and vitamin E, and an amino acid such as lysine, methionine, and histidine, a pigment such as β-carotene, astaxanthin, and canthaxanthin, a mineral such as calcium and silicate, a trace metal, and a preservative.

The feed for decapods may have any shape and size, depending on the type and size or the like of the decapod to be reared. For example, the feed for decapods may be a powdery feed obtained by mixing and pulverizing dry raw materials, a solidified feed (dry pellets) obtained by solidifying a powder, or a pasty feed containing moisture (moist pellets). In an example of methods for producing the feed for decapods, first, a common powdery feed for shrimp aquaculture and the lactic acid bacterial cell preparation are mixed together. This mixture is molded, for example, extrusion-molded by using a pasta machine or a syringe. Finally, the molded product is dried, for example, at 60°C to 65°C for about 2 hours, giving a feed for decapods containing the lactic acid bacterial cell preparation. The bacterial cell preparations of NITE-BP-03199, NITE-BP-03200, and NITE-BP-03201 do not lose their antibacterial activities to *Vibrio* bacteria even after being dried at high temperature. Other examples of the feed for decapods include a product obtained by dredging the lactic acid bacterial cell preparation over a common powdery feed for shrimp aquaculture and a product obtained by soaking a powdery feed for shrimp aquaculture in a liquid containing the lactic acid bacterial cell preparation (e.g., culture supernatant).

The amount of the lactic acid bacterial cell preparation contained in the feed for decapods is not limited as long as the antibacterial activity to *Vibrio* bacteria is allowed to be exhibited, and may be an amount suitable for the bacterial cell density of *Vibrio* bacteria, the rearing place, the rearing temperature, the oxygen concentration, the rearing density, the type of the decapod of interest, and so on. The total amount of the lactic acid bacterial cell preparation in the feed for decapods may be, for example, 1 × 10³ to 1 × 10¹² cfu (colony-forming unit)/g, or 1 × 10⁴ to 1 × 10¹² cfu/g, or 1 × 10⁵ to 1 × 10¹² cfu/g. The colony-forming unit can be determined by an agar plate culture method. The total amount of the lactic acid bacterial cell preparation (content ratio) in the feed for decapods may be 0.001% by mass to 50% by mass, preferably 0.001% by mass to 30% by mass, more preferably 0.01% by mass to 30% by mass, and even more preferably 0.01% by mass to 10% by mass to the feed weight. The total amount can be measured by weighing with a balance or the like.

The additive for the feed for decapods may be any additive that can be added to common feeds for aquaculture for decapods, without limitation. The feed additive may be liquid or powder, and may be freeze-dried. The feed additive may be the lactic acid bacterial cell preparation as it is. The feed additive may contain a liquid, a spreader, or the like for making it easy to allow the lactic acid bacterial cell preparation to be adhered to, absorbed in, or mixed with a feed for decapods. It is preferable that the feed additive be added to a feed in such a manner that the total amount of the bacterial cell preparation contained in the feed falls within the range shown above.

The feed for decapods containing the lactic acid bacterial cell preparation may be fed once or in multiple portions per day. The feed containing the lactic acid bacterial cell preparation may be fed once every several days. An appropriate period can be selected for feeding the feed containing the lactic acid bacterial cell preparation, for example, depending on the type of the decapod of interest. The feed containing the lactic acid bacterial cell preparation may be continuously fed for the whole period of aquaculture (rearing), or fed only for part of the period. The "part of the period" may be, for example, 10% or more, 20% or more, 30% or more, 50% or more, 70% or more, or 90% or more of the whole period of rearing. In an aspect of the present embodiment, the upper limit value of the "part of the period" is not limited, and the part of the period may be, for example, less than 100% of the whole period of rearing, or 99% or less thereof. The period to feed the feed containing the lactic acid bacterial cell preparation may be, for example, 1 month to 4 months, or approximately 2 months. The feed containing the lactic acid bacterial cell preparation may be fed in cycles of continuing feeding and suspending each for any period.

The composition for rearing decapods can be given to an organism belonging to the order Decapoda. Examples of methods for giving the composition for rearing decapods to an organism belonging to the order Decapoda include a method of mixing water to rear an organism belonging to the order Decapoda and the composition for rearing decapods. Examples of the methods of mixing water to rear an organism belonging to the order Decapoda and the composition for rearing an organism belonging to the order Decapoda include a method of dropping the composition for rearing an organism belonging to the order Decapoda into water, a method of pouring water into a rearing tank in which the composition for rearing an organism belonging to the order Decapoda is placed, and a method of adding the composition for rearing an organism belonging to the order Decapoda, dissolved in water, to water to rear an organism belonging to the order Decapoda. The composition for rearing an organism belonging to the order Decapoda may be automatically given to an organism belonging to the order Decapoda at constant intervals. For example, the feed for decapods may be provided by dropping the feed into water to rear an organism belonging to the order Decapoda, and may be automatically provided at constant intervals.

Each of the compositions for rearing an organism belonging to the order Decapoda may be a composition for soaking administration. The composition for soaking administration may be rearing water or a rearing water additive. The rearing water may be a liquid that is commonly used for rearing organisms belonging to the order Decapoda, and may be freshwater, brackish water, or seawater (including artificially prepared seawater). Rearing of an organism belonging to the order Decapoda may be performed in a natural environment, in an aquaculture pond, or in an aquarium. The rearing water may be aerated. The rearing water, which contains the lactic acid bacterial cell preparation, has antibacterial activity to *Vibrio* bacteria. Organisms belonging to the order Decapoda soaked in the rearing water can be prevented from being affected by infections caused by *Vibrio* bacteria.

The lactic acid bacterial cell preparation content in the rearing water is not limited as long as the antibacterial activity to *Vibrio* bacteria is allowed to be exhibited, and may be an amount suitable for the bacterial cell density of *Vibrio* bacteria, the rearing place, the rearing temperature, the oxygen concentration, the rearing density, the type of the decapod of interest, and so on. The total amount (content) of the lactic acid bacterial cell preparation in the rearing water may be, for example, 1 × 10² to 1 × 10⁸ cfu/mL, or 1 × 10³ to 1 × 10⁸ cfu/mL, or 1 × 10⁴ to 1 × 10⁸ cfu/mL. The colony-forming unit can be determined by an agar plate culture method. The total concentration of the lactic acid bacterial cell preparation contained in the rearing water is not limited, and may be 0.1 to 100,000 ppm, or 1 to 100,000 ppm, or 10 to 100,000 ppm. The total amount of the lactic acid bacterial cell preparation can be measured by weighing with a balance or the like. The concentration can be calculated from the mass ratio to the rearing water on the basis of the total amount determined.

The rearing water additive may be an additive that can be added to common rearing water for organisms belonging to the order Decapoda, without limitation. The rearing water additive may be liquid or powder, and may be freeze-dried. The rearing water additive may be the lactic acid bacterial cell preparation as it is. It is preferable that the rearing water additive be added to rearing water in such a manner that the total amount of the bacterial cell preparation contained in the rearing water falls within the range shown above.

The rearing water additive may be added to rearing water before the beginning of rearing of an organism belonging to the order Decapoda, or added to rearing water in which an organism belonging to the order Decapoda is reared. Examples of methods for adding the rearing water additive to rearing water include the aforementioned method of mixing water to rear an organism belonging to the order Decapoda and any of the compositions for rearing an organism belonging to the order Decapoda. For example, the rearing water additive may be provided by dropping the rearing water additive into water to rear an organism belonging to the order Decapoda, and may be automatically provided at constant intervals.

An appropriate period can be selected for soaking an organism belonging to the order Decapoda in the rearing water containing the lactic acid bacterial cell preparation. An organism belonging to the order Decapoda may be reared in the rearing water containing the lactic acid bacterial preparation continuously over the whole period to rear the organism belonging to the order Decapoda, or the organism belonging to the order Decapoda may be soaked in the rearing water only for part of the period. The period to rear in rearing water containing the lactic acid bacterial cell preparation may be, for example, 1 month to 4 months, or approximately 2 months. Rearing in the rearing water containing the lactic acid bacterial cell preparation may be performed in cycles of continuing and suspending each for any period.

An embodiment of the present invention is use of bacterial cells or a cell culture product of at least one bacterium selected from NITE-BP-03199, NITE-BP-03200, and NITE-BP-03201, or an extract thereof in production of a composition for rearing an organism belonging to the order Decapoda.

### [Method for rearing organism belonging to order Decapoda]

A method for rearing an organism belonging to the order Decapoda according to an embodiment of the present invention includes administering bacterial cells or a cell culture product of at least one bacterium selected from NITE-BP-03199, NITE-BP-03200, and NITE-BP-03201, or an extract thereof to an organism belonging to the order Decapoda. The administering may be oral administration or soaking administration.

A method for rearing an organism belonging to the order Decapoda according to an embodiment of the present invention includes allowing an organism belonging to the order Decapoda to ingest bacterial cells or a cell culture product of at least one bacterium selected from NITE-BP-03199, NITE-BP-03200, and NITE-BP-03201, or an extract thereof. The lactic acid bacterial cell preparation may be provided as the composition for rearing an organism belonging to the order Decapoda.

A method for rearing an organism belonging to the order Decapoda according to an embodiment of the present invention includes soaking an organism belonging to the order Decapoda in rearing water containing bacterial cells or a cell culture product of at least one bacterium selected from NITE-BP-03199, NITE-BP-03200, and NITE-BP-03201, or an extract thereof. The rearing water containing the lactic acid bacterial cell preparation may be provided as the composition for rearing an organism belonging to the order Decapoda.

In each of the methods for rearing an organism belonging to the order Decapoda, the composition for rearing an organism belonging to the order Decapoda may be given according to the aforementioned method of mixing water to rear an organism belonging to the order Decapoda and the composition for rearing an organism belonging to the order Decapoda. For example, in each of the methods for rearing an organism belonging to the order Decapoda, the composition for rearing an organism belonging to the order Decapoda may be provided by dropping the composition into water to rear an organism belonging to the order Decapoda, and may be automatically provided at constant intervals.

An embodiment of the present invention is use of bacterial cells or a cell culture product of at least one bacterium selected from NITE-BP-03199, NITE-BP-03200, and NITE-BP-03201, or an extract thereof in rearing an organism belonging to the order Decapoda.

### [Composition for preventing or treating infection caused by Vibrio bacterium in decapod]

A composition for preventing or treating an infection caused by a *Vibrio* bacterium in a decapod according to an embodiment of the present invention contains bacterial cells or a cell culture product of at least one bacterium selected from NITE-BP-03199, NITE-BP-03200, and NITE-BP-03201, or an extract thereof. The preventive or therapeutic composition may be a medicament for animals.

NITE-BP-03199, NITE-BP-03200, and NITE-BP-03201 each have antibacterial activity to *Vibrio* bacteria, and are capable of inhibiting the growth of *Vibrio* bacteria or killing *Vibrio* bacteria. Infections caused by *Vibrio* bacteria in decapods can be prevented or treated with NITE-BP-03199, NITE-BP-03200, and NITE-BP-03201. Herein, treatment includes mitigation of a symptom and improvement in and complete cure of a symptom. The lactic acid bacteria each have direct antibacterial activity to causative bacteria for infections, with the mechanism for inhibiting infections being independent of hosts, and thus are capable of inhibiting infections in decapods in a more universal manner, irrespective of the type or growth stage of the decapod of interest. Use of the lactic acid bacteria enables more stable aquaculture of organisms belonging to the order Decapoda.

Examples of infections caused by *Vibrio* bacteria in decapods include EMS/AHPND for which *Vibrio parahaemolyticus* is a causative bacterium.

Outbreaks of EMS/AHPND in juvenile shrimp cause mortality rates close to 100%, thus posing a critical problem in aquaculture industries. *Vibrio parahaemolyticus* that causes EMS/AHPND in organisms belonging to the order Decapoda is a bacterium being the same species as but of different type from *Vibrio parahaemolyticus* that is a causative bacterium to cause food poisoning in humans. Examples of other *Vibrio* bacteria that cause infections in organisms belonging to the order Decapoda include *V*. *nigripulchritudo, V*. *penaeicida, V*. *alginolyticus), V*. *anguillarum, V*. *campbelli, V*. *damsella, V*. *fischeri, V*. *harveyi, V*. *logei, V*. *mediterrani, V*. *ordalii, V*. *orientalis, V*. *pelagicus, V*. *splendidus, V*. *vulnificus, V*. *fluvialis,* and *V*. *cholerae.* Massive death of organisms belonging to the order Decapoda due to infections caused by *V*. *nigripulchritudo* and *V*. *penaeicida* has been also reported.

Each of the preventive or therapeutic compositions may be a composition for oral administration, and may be administered in the form of a feed or feed additive to an organism belonging to the order Decapoda. The preventive or therapeutic composition may contain a component that can be contained in the composition for oral administration to be used for rearing an organism belonging to the order Decapoda. The preventive or therapeutic composition may further contain a known component having antibacterial activity to *Vibrio* bacteria or component that increases the immunoreactivity of the organism belonging to the order Decapoda. The same shapes, production methods, and administration methods as for the composition for oral administration may be applied to the preventive or therapeutic composition. The lactic acid bacterial cell preparation content of the preventive or therapeutic composition is not limited as long as the content is such that infections caused by *Vibrio* bacteria can be inhibited in an organism belonging to the order Decapoda to which the composition has been administered, and may be in the same content range as in the composition for oral administration.

Each of the preventive or therapeutic compositions may be rearing water or a rearing water additive. The preventive or therapeutic composition as rearing water or a rearing water additive may be used or added in the same manner as rearing water or rearing water additives to be used for rearing organisms belonging to the order Decapoda. The lactic acid bacterial cell preparation content of the preventive or therapeutic composition is not limited as long as the content is such that infections caused by *Vibrio* bacteria can be inhibited in an organism belonging to the order Decapoda which has been soaked in the composition, and may be in the same content range as in the rearing water.

An embodiment of the present invention is use of a bacterial cell or cell culture product of at least one bacterium selected from NITE-BP-03199, NITE-BP-03200, and NITE-BP-03201, or an extract from the bacterial cell or cell culture product in production of a composition for preventing or treating an infection caused by a *Vibrio* bacterium in a decapod.

### [Method for preventing or treating infection caused by Vibrio bacterium in decapod]

A method for preventing or treating an infection caused by a *Vibrio* bacterium in a decapod according to an embodiment of the present invention includes administering bacterial cells or a cell culture product of at least one bacterium selected from NITE-BP-03199, NITE-BP-03200, and NITE-BP-03201, or an extract thereof to an organism belonging to the order Decapoda. The administering may be oral administration or soaking administration.

A method for preventing or treating an infection caused by a *Vibrio* bacterium in a decapod according to an embodiment of the present invention includes allowing an organism belonging to the order Decapoda to ingest bacterial cells or a cell culture product of at least one bacterium selected from NITE-BP-03199, NITE-BP-03200, and NITE-BP-03201, or an extract thereof. The lactic acid bacterial cell preparation may be provided as the preventive or therapeutic composition to an organism belonging to the order Decapoda.

A method for preventing or treating an infection caused by a *Vibrio* bacterium in a decapod according to an embodiment of the present invention includes soaking an organism belonging to the order Decapoda in rearing water containing bacterial cells or a cell culture product of at least one bacterium selected from NITE-BP-03199, NITE-BP-03200, and NITE-BP-03201, or an extract thereof. The rearing water containing the lactic acid bacterial cell preparation may be provided as any of the preventive or therapeutic compositions.

An embodiment of the present invention is use of bacterial cells or a cell culture product of at least one bacterium selected from NITE-BP-03199, NITE-BP-03200, and NITE-BP-03201, or an extract thereof for preventing or treating an infection caused by a *Vibrio* bacterium in a decapod.

### [Growth inhibitor or a bactericide, and growth-inhibiting or bactericidal method for Vibrio bacterium]

A growth inhibitor or a bactericide for a *Vibrio* bacterium according to an embodiment of the present invention contains bacterial cells or a cell culture product of at least one bacterium selected from NITE-BP-03199, NITE-BP-03200, and NITE-BP-03201, or an extract thereof. The same material as the composition for rearing an organism belonging to the order Decapoda can be used for the growth inhibitor or a bactericide. The growth inhibitor or a bactericide may further contain a substance having antibacterial activity to *Vibrio* bacteria.

The lactic acid bacterial cell preparation content of the growth inhibitor or the bactericide for *Vibrio* bacteria is not limited, and may be, for example, 1 × 10³ to 1 × 10¹² cfu/mL, or 1 × 10⁵ to 1 × 10¹² cfu/mL, or 1 × 10⁷ to 1 × 10¹² cfu/mL. The concentration of the lactic acid bacterial cell preparation contained in the growth inhibitor or the bactericide for *Vibrio* bacteria is not limited, and may be 1 to 1,000,000 ppm, or 100 to 1,000,000 ppm, or 10,000 to 1,000,000 ppm.

The lactic acid bacterial cell preparation is capable of inhibiting the growth of or killing *Vibrio* bacteria, and preferably inhibits the growth of or kills at least one selected from *V*. *parahaemolyticus, V*. *nigripulchritudo,* and *V*. *penaeicida.*

A growth-inhibiting or bactericidal method for a *Vibrio* bacterium according to an embodiment of the present invention includes bringing bacterial cells or a cell culture product of at least one bacterium selected from NITE-BP-03199, NITE-BP-03200, and NITE-BP-03201, or an extract thereof into contact with a *Vibrio* bacterium. Any method of bringing the lactic acid bacterial cell preparation into contact with a *Vibrio* bacterium is applicable without limitation, and, for example, the lactic acid bacterial cell preparation may be added to water in which a *Vibrio* bacterium is present, an object with the presence of a *Vibrio* bacterium may be soaked in a liquid containing the lactic acid bacterial cell preparation, and a host infected with a *Vibrio* bacterium may be allowed to ingest a composition containing the lactic acid bacterial cell preparation. The lactic acid bacterial cell preparation may be used in the form of any of the growth inhibitor or the bactericide for *Vibrio* bacteria.

An embodiment of the present invention is use of bacterial cells or a cell culture product of at least one bacterium selected from NITE-BP-03199, NITE-BP-03200, and NITE-BP-03201, or an extract thereof in production of a growth inhibitor or a bactericide for a *Vibrio* bacterium.

An embodiment of the present invention is use of bacterial cells or a cell culture product of at least one bacterium selected from NITE-BP-03199, NITE-BP-03200, and NITE-BP-03201, or an extract thereof for inhibiting the growth of or killing a *Vibrio* bacterium.

### EXAMPLES

Hereinafter, the present invention will be described in more detail with reference to Examples, but the present invention is not limited to those Examples.

### [Experiment 1: Isolation and identification of NITE BP-03199]

A source for isolation (wax apple) was ground together with sterile water. The solution given by grinding was appropriately diluted and added to 1/2 MRS liquid medium, and subjected to enrichment culture. The enrichment culture solution was smeared on MRS agar medium containing calcium carbonate, and a microorganism that formed a halo was isolated. Hereinafter, this isolate is referred to as isolate A. No bubble was generated when the culture solution of isolate A was suspended in hydrogen peroxide. From the absence of catalase, isolate A was confirmed to be a lactic acid bacterium.

Isolate A was identified through 16S rRNA gene analysis, morphological observation, and physiological/biochemical characteristics test.

### (1) 16S rRNA gene analysis

Genomic DNA was extracted from isolate A, and PCR amplification of a 16S rRNA gene was performed by using the extracted genomic DNA as a template with a forward primer for cloning, 9F, and a reverse primer for cloning, 1510R (NAKAGAWA Yasuyoshi et al.: Gene Analysis Method - Nucleotide Sequence Determination Method for 16S rRNA Gene, edited by The Society for Actinomycetes Japan, Identification Manual of Actinomycetes, pp. 88-117, Business Center for Academic Societies Japan, 2001). Tks Gflex DNA polymerase (manufactured by Takara Bio Inc.) was used in the PCR amplification performed, and a PCR amplification product was purified.

Cycle sequence reaction was performed with the purified PCR amplification product. A BigDye Terminator v3.1 Cycle Sequencing Kit was used in the cycle sequence reaction performed. The resulting reaction solution was purified, and the purified solution was subjected to DNA sequence analysis (3130xl DNA Analyzer) to determine the nucleotide sequence of the 16S rRNA gene of the template DNA extracted from isolate A. Primers used for the sequence analysis were 9F, 515F, 1099F, 536R, 926R, and 1510R (NAKAGAWA Yasuyoshi et al.: Gene Analysis Method - Nucleotide Sequence Determination Method for 16S rRNA Gene, edited by The Society for Actinomycetes Japan, Identification Manual of Actinomycetes, pp. 88-117, Business Center for Academic Societies Japan, 2001).

With the microorganism identification system "ENKI" (manufactured by TechnoSuruga Laboratory Co., Ltd.), the nucleotide sequence of the 16S rRNA gene of isolate A was subjected to BLAST homology search for the microorganism identification database DB-BA15.0 (established by TechnoSuruga Laboratory Co., Ltd.) and international nucleotide sequence databases (DDBJ/ENA(EMBL)/GenBank). The nucleotide sequence of the 16S rRNA gene of isolate A exhibited 100.0% identity with the nucleotide sequence of the 16S rRNA gene of *Lactobacillus pentosus* (JCM1558), 100.0% identity with the nucleotide sequence of the 16S rRNA gene of *Lactobacillus plantarum* subsp. *plantarum* (JCM1149), and 99.80% identity with the nucleotide sequence of the 16S rRNA gene of *Lactobacillus paraplantarum* (DSM10667).

### (2) Morphological observation and physiological/biochemical characteristics test

Isolated bacterium A was applied onto MRS agar medium, and subjected to aerobic culture at a temperature of 30°C for 48 hours, and the cell morphology, Gram stainability, mobility, and colony morphology were observed by the following methods.

The colony morphology was observed with the stereomicroscope SMZ800N (manufactured by Nikon Corporation). The cell morphology was observed with the optical microscope BX50F4 (manufactured by Olympus Corporation). In the Gram staining, Favor G "NISSUI" (manufactured by Nissui Pharmaceutical Co., Ltd.) was used. According to methods described in Barrow & Feltham (Cowan and Steel's Manual for the Identification of Medical Bacteria, 3rd ed. Cambridge: Cambridge University Press; 1993.), test was performed on catalase reaction, oxidase reaction, acid/gas production from glucose, and oxidation/fermentation (O/F) of glucose. The physiological/biochemical characteristics reaction of the bacterium was examined by using an API50CHB kit (manufactured by bioMerieux, France).

As shown in Fig. 1(A), isolate A formed circular colonies. As shown in Fig. 1(B), isolate A was positive for Gram stainability. Table 1 and Table 2 show results of the physiological/biochemical characteristics test and fermentability test for isolate A. Isolate A was a Gram-positive bacillus without mobility, formed no spore, exhibited negativity for catalase reaction and oxidase reaction, and fermented glucose. These characteristics were consistent with those of the genus *Lactobacillus,* to which isolate A was expected to belong as shown by the results of the 16S rDNA partial nucleotide sequence analysis. Results of the fermentability test performed with an API kit showed that isolate A fermented galactose, fructose, α-methyl-D-mannoside, melicitose, etc., and did not ferment glycerol, D-xylose, etc. Isolate A exhibited no arginine dihydrolase activity, and grew at 15°C. These characteristics were different from those of *L. pentosus* in that no fermentation of glycerol and D-xylose was caused and consistent with those of *L. plantarum,* wherein *L. pentosus* and *L. plantarum* were the species to either one of which isolate A was expected to belong as suggested by the results of the 16S rDNA partial nucleotide sequence analysis. Accordingly, isolate A was found to be a novel isolate belonging to *Lactobacillus plantarum.* Isolate A was internationally deposited as NITE BP-03199.

**[Table 1]**

| Test item | | NITE BP-03199 |
|---|---|---|
| Culture temperature | | 30°C |
| Cell morphology | | bacillus (0.7-0.8×2.0-3.0 µm) |
| Gram stainability | | + |
| Presence or absence of spore | | - |
| Mobility | | - |
| Colony morphology | Medium | MRS agar medium |
| | Culture time | 48 hours |
| | Diameter | 1 to 3 mm |
| | Color | milky white |
| | Shape | circular |
| | Elevation | lens-like |
| | Margin | entire |
| | Surface shape, etc. | smooth |
| | Transparency | opaque |
| | Viscosity | butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose | | +/- |
| (acid production/gas production) | | |
| O/F test | | + / + |
| (oxidation/fermentation) | | |

| | | |
|---|---|---|
| +: positive, -: negative | | |

**[Table 2]**

| Item | Substrate component | Test result | Item | Substrate component | Test result |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | |
| 9 | β-Methyl-D-xylose | - | 34 | Melicitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | + | 45 | D-Arabitol | - |
| 21 | α-Methyl-D-glucoside | + | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |

| | | | | | |
|---|---|---|---|---|---|
| +: positive, -: negative | | | | | |

### [Experiment 2: Isolation and identification of NITE BP-03200]

Isolate B was isolated in the same manner as in Experiment 1 except that *Pandanus odoratissimus* was used as a source for isolation. Identification of isolate B was performed in the same manner as in Experiment 1.

The nucleotide sequence of the 16S rRNA gene of isolate B exhibited 99.87% identity with the nucleotide sequence of the 16S rRNA gene of *Lactobacillus pentosus* (JCM1558), 99.87% identity with the nucleotide sequence of the 16S rRNA gene of *Lactobacillus plantarum* subsp. *plantarum* (JCM1149), and 99.66% identity with the nucleotide sequence of the 16S rRNA gene of *Lactobacillus paraplantarum* (DSM10667). However, there was no microorganism having a 16S rRNA gene the nucleotide sequence of which completely matched with that of the 16S rRNA gene of isolate B.

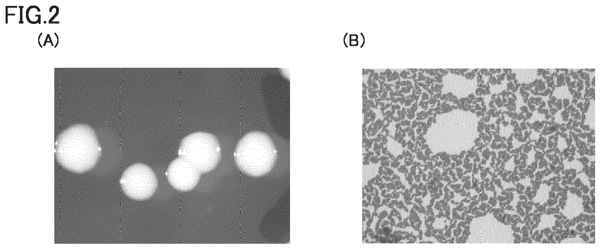

As shown in Fig. 2(A), isolate B formed circular colonies. As shown in Fig. 2(B), isolate B was positive for Gram stainability. Table 3 and Table 4 show results of the physiological/biochemical characteristics test and fermentability test for isolate B. Isolate B was a Gram-positive bacillus without mobility, formed no spore, exhibited negativity for catalase reaction and oxidase reaction, and fermented glucose. These characteristics were consistent with those of the genus *Lactobacillus,* to which isolate B was expected to belong as shown by the results of the 16S rDNA partial nucleotide sequence analysis. Results of the fermentability test performed with an API kit showed that isolate B fermented galactose, fructose, α-methyl-D-mannoside, melicitose, etc., and did not ferment glycerol, D-xylose, etc. Isolate B exhibited no arginine dihydrolase activity, and grew at 15°C. These characteristics were different from those of *L. pentosus* in that no fermentation of glycerol and D-xylose was caused and consistent with those of *L. plantarum,* wherein *L. pentosus* and *L. plantarum* were the species demonstrated to be relatives to isolate B by the results of the 16S rDNA partial nucleotide sequence analysis. Accordingly, isolate B was found to be a novel isolate belonging to *Lactobacillus plantarum.* Isolate B was internationally deposited as NITE BP-03200.

**[Table 3]**

| Test item | | NITE BP-03200 |
|---|---|---|
| Culture temperature | | 30°C |
| Cell morphology | | bacillus (0.7-0.8×2.0-3.0 µm) |
| Gram stainability | | + |
| Presence or absence of spore | | - |
| Mobility | | - |
| Colony morphology | Medium | MRS agar medium |
| | Culture time | 48 hours |
| | Diameter | 1 to 3 mm |
| | Color | milky white |
| | Shape | circular |
| | Elevation | lens-like |
| | Margin | entire |
| | Surface shape, etc. | smooth |
| | Transparency | opaque |
| | Viscosity | butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose | | +/- |
| (acid production/gas production) | | |
| O/F test | | +/+ |
| (oxidation/fermentation) | | |

| | | |
|---|---|---|
| +: positive, -: negative | | |

**[Table 4]**

| Item | Substrate component | Test result | Item | Substrate component | Test result |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | - | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | |
| 9 | β-Methyl-D-xylose | - | 34 | Melicitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | + | 45 | D-Arabitol | - |
| 21 | α-Methyl-D-glucoside | - | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |

| | | | | | |
|---|---|---|---|---|---|
| +: positive, -: negative | | | | | |

### [Experiment 3: Isolation and identification of NITE BP-03201]

Isolate C was isolated in the same manner as in Experiment 1 except that *Ficus variegata Blume* was used as a source for isolation. Identification of isolate C was performed in the same manner as in Experiment 1.

The nucleotide sequence of the 16S rRNA gene of isolate C exhibited 99.93% identity with the nucleotide sequence of the 16S rRNA gene of *Lactobacillus pentosus* (JCM1558), 99.93% identity with the nucleotide sequence of the 16S rRNA gene of *Lactobacillus plantarum* subsp. *plantarum* (JCM1149), and 99.73% identity with the nucleotide sequence of the 16S rRNA gene of *Lactobacillus paraplantarum* (DSM10667). However, there was no microorganism having a 16S rRNA gene the nucleotide sequence of which completely matched with that of the 16S rRNA gene of isolate C.

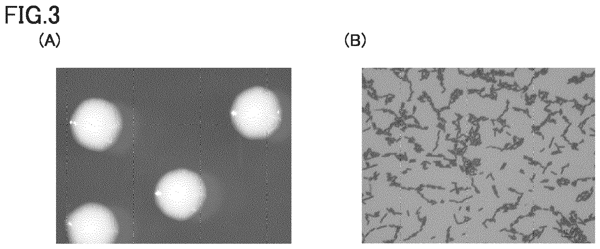

As shown in Fig. 3(A), isolate C formed circular colonies. As shown in Fig. 3(B), isolate C was positive for Gram stainability. Table 5 and Table 6 show results of the physiological/biochemical characteristics test and fermentability test for isolate C. Isolate C was a Gram-positive bacillus without mobility, formed no spore, exhibited negativity for catalase reaction and oxidase reaction, and fermented glucose. These characteristics were consistent with those of the genus *Lactobacillus,* to which isolate C was expected to belong as shown by the results of the 16S rDNA partial nucleotide sequence analysis. Results of the fermentability test performed with an API kit showed that isolate C fermented galactose, fructose, melicitose, etc., and did not ferment glycerol, D-xylose, etc. Isolate C exhibited no arginine dihydrolase activity, and grew at 15°C. These characteristics were different from those of *L. pentosus* in that no fermentation of glycerol and D-xylose was caused and consistent with those of *L. plantarum,* wherein *L. pentosus* and *L. plantarum* were the species to either one of which isolate C was expected to belong as suggested by the results of the 16S rDNA partial nucleotide sequence analysis. Accordingly, isolate C was found to be a novel isolate belonging to *Lactobacillus plantarum.* Isolate C was internationally deposited as NITE BP-03201.

**[Table 5]**

| Test item | | NITE BP-03201 |
|---|---|---|
| Culture temperature | | 30°C |
| Cell morphology | | bacillus (0.9-1.0×2.0-4.0 µm) |
| Gram stainability | | + |
| Presence or absence of spore | | - |
| Mobility | | - |
| Colony morphology | Medium | MRS agar medium |
| | Culture time | 48 hours |
| | Diameter | 1 to 3 mm |
| | Color | milky white |
| | Shape | circular |
| | Elevation | lens-like |
| | Margin | entire |
| | Surface shape, etc. | smooth |
| | Transparency | opaque |
| | Viscosity | butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (acid production/gas production) | | + / - |
| O/F test (oxidation/fermentation) | | + / + |

| | | |
|---|---|---|
| +: positive, -: negative | | |

**[Table 6]**

| Item | Substrate component | Test result | Item | Substrate component | Test result |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | |
| 9 | β-Methyl-D-xylose | - | 34 | Melicitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | - | 45 | D-Arabitol | - |
| 21 | α-Methyl-D-glucoside | - | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |

| | | | | | |
|---|---|---|---|---|---|
| +: positive, -: negative | | | | | |

### [Experiment 4: Test for evaluating antibacterial activities of lactic acid cell culture products (plate culture)]

Examination was performed on whether the cell culture products of NITE BP-03199, NITE BP-03200, and NITE BP-03201 have antibacterial activity to *Vibrio* bacteria. The *V*. *parahaemolyticus* TUMSAT-D6 strain (AHPND strain), *V*. *nigripulchritudo* TUMSAT-OK1 strain, and *V*. *penaeicida* TUMSAT-OG1 strain were used as *Vibrio* bacteria.

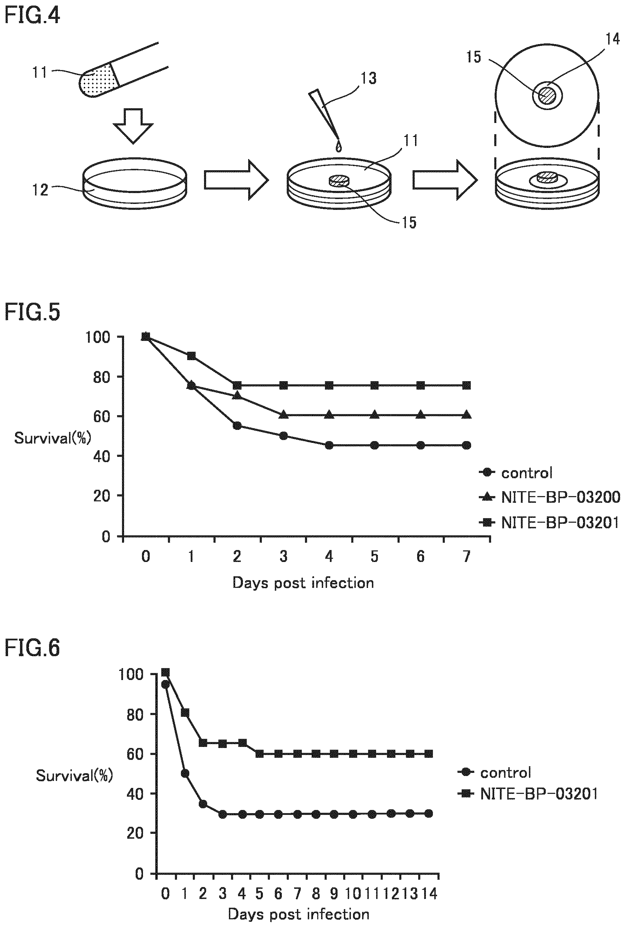

With reference to Fig. 4, the experimental method will be described. With a bacteria spreader, 100 µL of a bacterial solution 11 of a *Vibrio* bacterium (approximately 10⁸ cfu/mL) was smeared on agar medium 12. The agar medium used was Marine agar (Difco). A filter paper disk 15 was impregnated with 20 µL of a lactic acid bacterial culture filtrate 13, and placed on the agar medium with a *Vibrio* bacterium smeared thereon. The lactic acid bacterial culture filtrate (cell culture product) was a solution obtained through a process that any lactic acid bacterium of NITE BP-03199, NITE BP-03200, and NITE BP-03201 was cultured with MRS Broth at a temperature of 30°C and the culture supernatant was filtered through a filter having a pore size of 0.22 µm. The turbidity of each lactic acid bacterial culture solution at a wavelength of 600 nm was approximately 10 OD. The agar medium with filter paper disk 15 placed thereon was subjected to culture at 25°C for 24 hours, and the diameter (mm) of a growth-inhibitory zone 14 was measured. The results are shown in Table 7. Each numerical value shown in Table 7 indicates the diameter (mm) of the growth-inhibitory zone. A filter paper disk was impregnated with medium for lactic acid bacteria (MRS Broth) as a negative control, and another one was impregnated with the antibiotic ampicillin (100 µg/mL) as a positive control.

**[Table 7]**

| | NITE-BP-03199 | NITE-BP-03200 | NITE-BP-03201 | MRS Broth | Ampicillin |
|---|---|---|---|---|---|
| *Vibrio parahaemolyticus* | 10 | 10 | 10 | 0 | 28 |
| *Vibrio nigripulchritudo* | 14 | 13 | 16 | 0 | 29 |
| *Vibrio penaeicida* | 13 | 12 | 16 | 0 | 37 |

All the cell culture products of NITE BP-03199, NITE BP-03200, and NITE BP-03201 allowed the formation of a growth-inhibitory zone on the growth media for the *Vibrio* bacteria. It was demonstrated that all the cell culture products of NITE BP-03199, NITE BP-03200, and NITE BP-03201 are capable of inhibiting the growth of the *Vibrio* bacteria.

### [Experiment 5: Test for evaluating antibacterial activity of lactic acid cell culture product (liquid culture)]

Examination was performed on whether the cell culture product of NITE BP-03201, which exhibited the highest antibacterial activity in the plate culture method, is capable of inhibiting the growth of *Vibrio* bacteria even in liquid. Experiment 5 differed from Experiment 4 in that *Vibrio* bacteria were cultured in liquid media. The *Vibrio* bacterial strains used in Experiment 5 were the same as those in Experiment 4.

Liquid medium for *Vibrio* bacteria (3HI medium) was prepared by adding 2.5% NaCl to Heart Infusion broth (Difco) to reach an NaCl final concentration of 3%. A *Vibrio* bacterium was added to 3HI medium, and the bacterial solution was adjusted to approximately 10⁶ cfu/mL. To 450 µL of the bacterial solution, 50 µL of a culture filtrate of NITE BP-03201 cultured with MRS Broth was added and mixed therewith, and the resultant was cultured at 25°C for 18 hours. The culture filtrate of NITE BP-03201 had been prepared from a lactic acid bacterial culture solution having a turbidity of approximately 10 OD at a wavelength of 600 nm.

The bacterial cell count of each *Vibrio* bacterium was calculated from turbidity at a wavelength of 600 nm. In a sample with addition of MRS Broth alone (negative control), each *Vibrio* bacterium grown to an abundance approximately 1000 times higher than that before culture. In contrast to this, no growth was found for the *Vibrio* bacteria in the samples with addition of the culture filtrate of NITE BP-03201. The cell culture product of NITE BP-03201 was demonstrated to be capable of inhibiting the growth of the *Vibrio* bacteria even in liquid.

Furthermore, the following experiment was performed. A sample cultured with addition of the culture filtrate of NITE BP-03201 for 18 hours was centrifuged to remove the supernatant, and a collected bacterium *(Vibrio* bacterium) was resuspended in 3HI medium and additionally cultured for 24 hours. No growth was found for the bacteria after the culture. The result that no growth was found for the *Vibrio* bacteria even after removing the lactic acid cell culture product suggested that the cell culture product of NITE BP-03201 exhibits not bacteriostatic action but bactericidal action to the *Vibrio* bacteria.

### [Experiment 6: Test for evaluating antibacterial activities of lactic acid cell culture products (sterile seawater culture)]

Examination was performed on whether the cell culture products of NITE BP-03199, NITE BP-03200, and NITE BP-03201 have antibacterial activity to a *Vibrio* bacterium. Experiment 6 differed from Experiment 4 in that a *Vibrio* bacterium was cultured in artificial seawater. The *V*. *parahaemolyticus* TUMSAT-D6 strain (AHPND strain) was used as a *Vibrio* bacterium in Experiment 6.

The *Vibrio* bacterium cultured with 3HI medium (2 × 10⁹ cfu/mL) was suspended in artificial seawater to reach approximately 2 × 10² cfu/mL. The artificial seawater had been prepared by using Marine Salt (Tetra) and sterilized with a filter having a pore size of 0.22 µm. The artificial seawater containing the *Vibrio* bacterium was aliquoted into 450-µL, 475-µL, and 490-µL portions in 1.5-mL tubes, and 50 µL (1/10 dilution), 25 µL (1/20 dilution), and 10 µL (1/50 dilution) of a lactic acid bacterial culture filtrate of any of NITE BP-03199, NITE BP-03200, and NITE BP-03201 were respectively added thereto. As a negative control, MRS Broth, which is medium for lactic acid bacteria, was added. The mixed solutions were left to stand in an incubator at 25°C. After 1 hour, 3 hours, and 6 hours of culturing, a 50-µL portion was taken from each of the solutions in the tubes, smeared on 3HI agar medium, and cultured at 25°C overnight. Table 8 shows the numbers of colonies generated on the agar media.

**[Table 8]**

| | NITE-BP-03199 | | | NITE-BP-03200 | | | NITE-BP-03201 | | | MRS Broth | | |
|---|---|---|---|---|---|---|---|---|---|---|---|---|
| | 1h | 3h | 6h | 1h | 3h | 6h | 1h | 3h | 6h | 1h | 3h | 6h |
| 1/10 dilution | 3 | 0 | 0 | 2 | 0 | 0 | 3 | 0 | 0 | 1 | 8 | 198 |
| 1/20 dilution | 1 | 1 | 1 | 2 | 2 | 1 | 1 | 3 | 1 | 1 | 14 | >200 |
| 1/50 dilution | 4 | 2 | 4 | 4 | 5 | 26 | 2 | 5 | 146 | 2 | 8 | >200 |

As compared with the seawater with addition of MRS Broth (negative control), the growth of the *Vibrio* bacterium was significantly inhibited in all the seawaters with addition of a lactic acid bacterial culture filtrate. The cell culture products of NITE BP-03199, NITE BP-03200, and NITE BP-03201 were all demonstrated to be capable of inhibiting the growth of the *Vibrio* bacterium in seawater.

### [Experiment 7: Examination of preventive or therapeutic effect of oral administration of lactic acid bacterium for EMS/AHPND]

Examination was performed on whether an orally administered lactic acid cell culture product is capable of inhibiting an infection caused by a *Vibrio* bacterium in shrimp. The *V*. *parahaemolyticus* TUMSAT-D6 strain (AHPND strain) was used as a *Vibrio* bacterium. As a rearing environment for shrimp, 10 L of artificial seawater in a 18-L glass aquarium was prepared. Shrimp were reared while the rearing water was filtered through a SUISAKU EIGHT in M size. The water temperature was set to 28°C. To each test group, 20 vannamei shrimp were assigned.

Compositions for oral administration for rearing organisms belonging to the order Decapoda were each produced by adding a lactic acid bacterial culture filtrate to a commercially available granular feed that is used in Thailand to such a degree that the feed was sufficiently soaked in the lactic acid bacterial culture filtrate. The lactic acid bacteria used were NITE BP-03200 and NITE BP-03201. The lactic acid bacterial culture filtrates had been each prepared from a lactic acid bacterial culture solution having a turbidity of approximately 10 OD at a wavelength of 600 nm. A product obtained by adding MRS Broth, which is medium for lactic acid bacteria, to the feed was prepared as a negative control. Feeding was performed with a daily amount of about 10% of the body weight of shrimp in three or four separate portions. Feeding of each composition containing a lactic acid cell culture product for oral administration was initiated 3 days before infection test, and feeding was continued also during the infection test. The infection test was performed by adding 0.25 mL of a *Vibrio* bacterial solution at 2 × 10⁹ cfu/mL to the 10-L artificial seawater. Fig. 5 shows survival rates of shrimp from the initiation of infection.

Shrimp to which a composition containing a cell culture product of either one of NITE BP-03200 and NITE BP-03201 for oral administration for rearing organisms belonging to the order Decapoda exhibited higher survival rates than the control exhibited. It was demonstrated that oral administration of a cell culture product of any of NITE BP-03200 and NITE BP-03201 can prevent the infection caused by the *Vibrio* bacterium (EMS/AHPND).

### [Experiment 8: Examination of preventive or therapeutic effect of oral administration of lactic acid bacterium for EMS/AHPND]

A composition for oral administration for rearing organisms belonging to the order Decapoda was produced in a manner differing from that in Experiment 7. This was orally administered to shrimp to examine the infection-inhibiting effect. The rearing environment for shrimp and the *Vibrio* bacterium used were the same as those in Experiment 7.

A granular feed was pulverized, 24 mL of a culture filtrate of NITE BP-03201 was added to 20 g of the feed, which was kneaded and molded with a pasta machine, and the resultant was dried at 60°C for about 2 hours. The culture filtrate of NITE BP-03201 had been prepared from a lactic acid bacterial culture solution having a turbidity of approximately 10 OD at a wavelength of 600 nm. Feeding of the composition for oral administration was initiated 3 days before infection experiment, and feeding was continued also during the infection test. As a negative control, a common feed was fed to shrimp. The *Vibrio* bacterium was added to the artificial seawater to reach 2.6 × 10⁵ cfu/mL, shrimp were soaked therein for 6 hours, and the aerator was then exchanged. Fig. 6 shows the numbers of surviving individuals of shrimp for 14 days from the initiation of infection.

While the survival rate of shrimp in the group fed with the common feed was approximately 30%, the survival rate of shrimp in the group to which the composition containing the cell culture product of NITE BP-03201 for oral administration for rearing organisms belonging to the order Decapoda was 60%. The cell culture product of NITE BP-03201 was demonstrated not to lose the antibacterial activity to the *Vibrio* bacterium even after being subjected to high-temperature treatment and drying treatment, and to be capable of preventing or treating the infection caused by the *Vibrio* bacterium (EMS/AHPND) even through feeding with a composition containing the cell culture product after being dried.

### [Experiment 9: Examination of preventive or therapeutic effect of rearing water containing culture product of lactic acid bacterium for EMS/AHPND]

The cell culture products of NITE BP-03199, NITE BP-03200, and NITE BP-03201 were each added to rearing water, and examination was performed on whether an infection caused by a *Vibrio* bacterium can be inhibited in shrimp reared in such rearing water. The rearing environment for shrimp and the *Vibrio* bacterium used were the same as those in Experiment 7. For feeding, a commercially available granular feed that is used in Thailand was fed in the morning and in the evening.

Shrimp were put in the rearing aquarium, and 10 mL of a lactic acid bacterial culture filtrate of any of NITE BP-03199, NITE BP-03200, and NITE BP-03201, or MRS Broth, which is medium for lactic acid bacteria, as a negative control was added to the 10-L rearing water. The lactic acid bacterial culture filtrate had been prepared from a lactic acid bacterial culture solution having a turbidity of approximately 10 OD at a wavelength of 600 nm. After that, 0.25 mL of a bacterial solution of the *Vibrio* bacterium at 2 × 10⁹ cfu/mL was added to the rearing water. After the addition, aeration was performed with an air stone for 3 hours, and then the air stone was replaced with a filtration device (SUISAKU EIGHT in M size). Twenty-four hours after the initiation of infection with the *Vibrio* bacterium, 10 mL of the lactic acid bacterial culture filtrate was further added to the rearing water. Table 9 shows the numbers of surviving individuals of shrimp for 6 days from the initiation of infection.

**[Table 9]**

| Day | NITE-BP-03199 | NITE-BP-03200 | NITE-BP-03201 | MRS Broth |
|---|---|---|---|---|
| 0 | 20 | 20 | 20 | 20 |
| 1 | 20 | 20 | 19 | 17 |
| 2 | 20 | 20 | 19 | 17 |
| 3 | 20 | 20 | 19 | 17 |
| 4 | 20 | 20 | 19 | 16 |
| 5 | 20 | 20 | 19 | 14 |
| 6 | 20 | 20 | 19 | 14 |

The rearing waters with addition of a lactic acid bacterial culture filtrate of any of NITE BP-03199, NITE BP-03200, and NITE BP-03201 resulted in larger numbers of surviving individuals of shrimp than the control group (MRS Broth). It was demonstrated that rearing water containing a cell culture product of any of NITE BP-03199, NITE BP-03200, and NITE BP-03201 is useful for preventing the infection caused by the *Vibrio* bacterium (EMS/AHPND).

### REFERENCE SIGNS LIST

11 Bacterial solution of *Vibrio* bacterium; 12 Agar medium; 13 Lactic acid bacterial culture filtrate; 14 Growth-inhibitory zone; 15 Filter paper disk.

## Claims

1. A composition for rearing an organism belonging to the order Decapoda, comprising bacterial cells or a cell culture product of at least one bacterium selected from NITE-BP-03199, NITE-BP-03200, and NITE-BP-03201, or an extract thereof.

2. A composition for preventing or treating an infection caused by a *Vibrio* bacterium in a decapod, comprising bacterial cells or a cell culture product of at least one bacterium selected from NITE-BP-03199, NITE-BP-03200, and NITE-BP-03201, or an extract thereof.

3. The composition according to claim 2, wherein the infection in a decapod includes early mortality syndrome/acute hepatopancreatic necrosis disease.

4. The composition according to claim 1 or 2, wherein the composition is a feed or a feed additive.

5. The composition according to claim 1 or 2, wherein the composition is rearing water or a rearing water additive.

6. The composition according to claim 1 or 2, wherein the order Decapoda includes the family Penaeidae.

7. A method for rearing an organism belonging to the order Decapoda, comprising allowing the organism belonging to the order Decapoda to ingest bacterial cells or a cell culture product of at least one bacterium selected from NITE-BP-03199, NITE-BP-03200, and NITE-BP-03201, or an extract thereof.

8. A method for rearing an organism belonging to the order Decapoda, comprising soaking the organism belonging to the order Decapoda in rearing water containing bacterial cells or a cell culture product of at least one bacterium selected from NITE-BP-03199, NITE-BP-03200, and NITE-BP-03201, or an extract thereof.

9. A method for preventing or treating an infection caused by a *Vibrio* bacterium in a decapod, comprising allowing an organism belonging to the order Decapoda to ingest bacterial cells or a cell culture product of at least one bacterium selected from NITE-BP-03199, NITE-BP-03200, and NITE-BP-03201, or an extract thereof.

10. A method for preventing or treating an infection caused by a *Vibrio* bacterium in a decapod, comprising soaking an organism belonging to the order Decapoda in rearing water containing bacterial cells or a cell culture product of at least one bacterium selected from NITE-BP-03199, NITE-BP-03200, and NITE-BP-03201, or an extract thereof.

11. The method according to claim 9 or 10, wherein the infection in a decapod includes early mortality syndrome/acute hepatopancreatic necrosis disease.

12. The method according to any one of claims 7 to 10, wherein the order Decapoda includes the family Penaeidae.

13. A method for rearing an organism belonging to the order Decapoda, comprising providing the composition according to claim 1 or 2 by dropping the composition into water to rear the organism belonging to the order Decapoda.

14. A growth-inhibiting or bactericidal method for a *Vibrio* bacterium, comprising bringing bacterial cells or a cell culture product of at least one bacterium selected from NITE-BP-03199, NITE-BP-03200, and NITE-BP-03201, or an extract thereof into contact with the *Vibrio* bacterium.
